# EUROPEAN PATENT SPECIFICATION

(11) **EP 4 392 782 B1**
(45) Date of publication and mention of the grant of the patent: **28.10.2026**
(21) Application number: 22772808.6
(22) Date of filing: 26.08.2022
(51) Int. Cl.: G01N 33/68, G01N 27/447, G01N 33/84

(54) **EX VIVO ANALYTIC METHOD**
EX-VIVO-ANALYSEVERFAHREN
PROCÉDÉ D'ANALYSE EX VIVO

(30) Priority: 27.08.2021 EP 21193414
(43) Date of publication of application: 03.07.2024
(73) Proprietor: Helmholtz Zentrum München - Deutsches Forschungszentrum für Gesundheit und Umwelt (GmbH), 85764 Neuherberg (DE); Georg-August-Universität Göttingen Stiftung Öffentlichen Rechts, Universitätsmedizin, 37075 Göttingen (DE)
(72) Inventor: MICHALKE, Bernhard, 85570 Markt Schwaben (DE); VENKATARAMANI, Vivek, 97080 Würzburg (DE)
(74) Representative: Schiweck Weinzierl Koch Patentanwälte Partnerschaft mbB
(86) International application number: PCT/EP2022/073848
(87) International publication number: WO 2023/025957

(56) References cited:
- MICHALKE BERNHARD ET AL: "Iron Redox Speciation Analysis Using Capillary Electrophoresis Coupled to Inductively Coupled Plasma Mass Spectrometry (CE-ICP-MS)", FRONTIERS IN CHEMISTRY, vol. 7, 14 March 2019 (2019-03-14), XP055884954, DOI: 10.3389/fchem.2019.00136
- MICHALKE BERNHARD ET AL: "Setup of Capillary Electrophoresis-Inductively Coupled Plasma Mass Spectrometry (CE-ICP-MS) for Quantification of Iron Redox Species (Fe(II), Fe(III))", JOURNAL OF VISUALIZED EXPERIMENTS, no. 159, 4 May 2020 (2020-05-04), XP055884958, DOI: 10.3791/61055
- MICHALKE B ET AL: "Application of capillary zone electrophoresis-inductively coupled plasma mass spectrometry and capillary isoelectric focusing-inductively coupled plasma mass spectrometry for selenium speciation", JOURNAL OF CHROMATOGRAPHY A, ELSEVIER, AMSTERDAM, NL, vol. 807, no. 1, 20 May 1998 (1998-05-20), pages 71 - 80, XP004123625, ISSN: 0021-9673, DOI: 10.1016/S0021-9673(97)01231-4

## Description

### TECHNICAL FIELD OF THE INVENTION

The present invention relates to an ex vivo analytic method for analysis of a sample (S1), comprising the steps: A) Determining Fe²⁺, Fe³⁺, Fe-Ferritin and total Fe content in sample (S1), comprising the steps: A1) Separating simultaneously Fe²⁺, Fe³⁺ and Fe-Ferritin present in a sample (S1); and A2) Quantifying simultaneously Fe²⁺, Fe³⁺, Fe-Ferritin separated in step A1) and total Fe based on sample (S1); B) Determining S and Se containing compounds content of sample (S1), comprising the steps B1) Separating simultaneously S and Se containing compounds present in sample (S1); and B2) Quantifying simultaneously S and Se containing compounds separated in step B1) of sample (S1). Wherein the S containing compounds are selected from the group consisting of oxidized glutathione (GSSG), reduced glutathione (GSH), cystine, and/or cysteine; and the Se containing compounds are selected from Se⁴⁺, Se⁶⁺, Se-methionine, Se-cysteine GPX4, Selenoprotein P, and/or Seleno sugars.

Further, the invention relates to a kit for performing the analytic method.

### BACKGROUND ART

The pathogenesis of a couple of severe diseases is strongly connected to dysregulation and even derailment of the cellular redox balance, typically mediated by increased ferrous iron (Fe²⁺), decreased ferric iron (Fe³⁺) and changes in the ferritin iron pool. This dysregulation is further reflected in metabolic downstream by a shift in glutathione equilibrium (oxidized vs. reduced). The past two decades have revealed the existence of multiple distinct non-apoptotic cell death mechanisms that are associated with a variety of diseases [1]. These mechanisms include Ferroptosis (derived from Latin *ferro,* "ferrous iron" (Fe²⁺), and *ptosis,* from the Greek "to fall"), which is a newly discovered non-apoptotic cell death believed to be a major driver of common neurodegenerative conditions [2, 3], but also holds great promise for therapy-refractory cancer entities or even diabetes [4, 5]. This regulated necrotic cell death is a metabolic process of cell sabotage where disruption of the antioxidant machinery allows a toxic buildup of membrane-damaging lipid peroxides [2, 6].

The unique hallmarks of ferroptosis are determined by
i) unrestrained abundance of pro-oxidative Fe²⁺,
ii) Fe²⁺-mediated oxidation of membrane-bound polyunsaturated fatty acids (PUFAs), subsumed under the term lipid peroxidation, and
iii) the loss of lipid peroxidation repair enzymes, such as glutathione peroxidase 4 (GPX4), which in turn chemically has to be considered as a seleno-compound. Selenium has a non-interchangeable role in GPX4 and has a prominent function in suppressing ferroptosis [10]. GPX4 can directly detoxify peroxides using glutathione (GSH) as co-substrate [2, 7, 8]. The contribution of GSH depletion, ferritin-targeted autophagy, and other iron importing systems adds a layer of complexity to the temporal changes of the Fe²⁺-GSH complex in ferroptosis [4]. The molar GSH:GSSG ratio exceeds 100:1 in a resting cell, while under oxidative stress, this ratio has been demonstrated to decrease to values of 10:1 to even 1:1
Michalke B. et al., Fontiers in Chemistry 2019, vol. 7, disclose a iron redox speciation analysis capillary electrophoresis coupled to inductively coupled plasma mass spectrometry (CE-ICP-MS).
Michalke B. et al., Journal of Visualized Experiments 2020, no. 159, disclose a setup of capillary electrophoresis-inductively coupled plasma mass spectroscopy (CE-ICP-MS) for quantification of iron redox species (Fe(II), Fe(III).
Michalke B. et al., Journal of Chromatography A, 1998, vol. 807, no. 1, p. 71-80 disclose an application of capillary zone electrophoresis-inductively coupled plasma mass spectrometry and capillary isoelectric focusing-inductively coupled plasma masss spectrometry for selenium speciation.

In the prior art methods [9] are known to determine in a sample the content of some of the biological markers as dicussed above, such as Fe²⁺ and/or Fe³⁺, but there is no method yet to determine based on one sample several different factors of ferroptosis such as different iron, sulfur and/or selenium species simultaneously.

### SUMMARY OF THE INVENTION

In a first aspect, the invention relates to an ex vivo analytic method for analysis of a sample (S1), comprising the steps:
A) Determining Fe²⁺, Fe³⁺, Fe-Ferritin and total Fe content in sample (S1), comprising the steps:
   A1) Separating simultaneously Fe²⁺, Fe³⁺ and Fe-Ferritin present in a sample (S1);
   A2) Quantifying simultaneously Fe²⁺, Fe³⁺, Fe-Ferritin separated in step A1) and total Fe based on sample (S1);
B) Determining S and Se containing compounds content of sample (S1), comprising the steps B1) Separating simultaneously S and Se containing compounds present in sample (S1);
   B2) Quantifying simultaneously S and Se containing compounds separated in step B1) of sample (S1).

Wherein the S containing compounds are selected from the group consisting of oxidized glutathione (GSSG), reduced glutathione (GSH), cystine, and/or cysteine;
and the Se containing compounds are selected from Se⁴⁺, Se⁶⁺, Se-methionine, Se-cysteine GPX4, Selenoprotein P, and/or Seleno sugars.

In a second aspect, the invention relates to the use of the analytic method for an ex vivo diagnosis of oxidative stress.

In a third aspect, the invention relates to the use of the analytic method for an ex vivo diagnosis of ferroptosis.

In a fourth aspect, the invention relates to the use of the analytic method for an ex vivo diagnosis of ferroptosis-associated diseases.

In a fifth aspect, the invention relates to the use of a kit for performing the analytic method, comprising
i) devices for performing the analytic method; and
ii) a HCl solution, preferably with a concentration of 20 to 80 mM, more preferably 30 to 70 mM, most preferably 50 mM; for performing step A1) and
iii) a HCl solution, preferably with a concentration of 1 to 15 mM, more preferably 3 to 8 mM, most preferably 5 mM; for performing step A1) and
iv) CH₄ gas for performing step B2).

The present inventive analytic method allows quantitative and parallel determination of Fe²⁺, Fe³⁺, Fe-bound ferritin and total Fe out of the same sample. In preferred embodiments in a short time, such as 5 to 15 min. Moreover, the invention allows quantitative and parallel determination of for example GSH, GSSG, cysteine, Se⁴⁺, Se⁶⁺, Se-methionine, cystine, Se-cystine and GPX4-n. The inventive analytical method comprises in one embodiment a "one-pot, two-shot" CE-ICP-DRC-MS approach which accomplishes an analytical coverage of nearly all ferroptosis-relevant information, such as the biomarkers as discussed above, in many biofluids, including cell and tissue lysates, CSF, serum, urine at a fraction of cost, empowering researchers to capture metallomic and redox-biological information that is infeasible to access otherwise using methods known in the prior art.

### BRIEF DESCRIPTION OF THE FIGURES

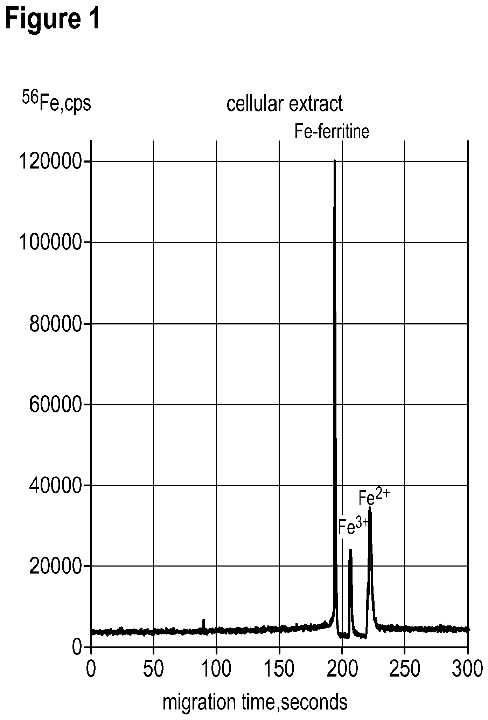

**Fig. 1**: Iron speciation in a cellular extract.

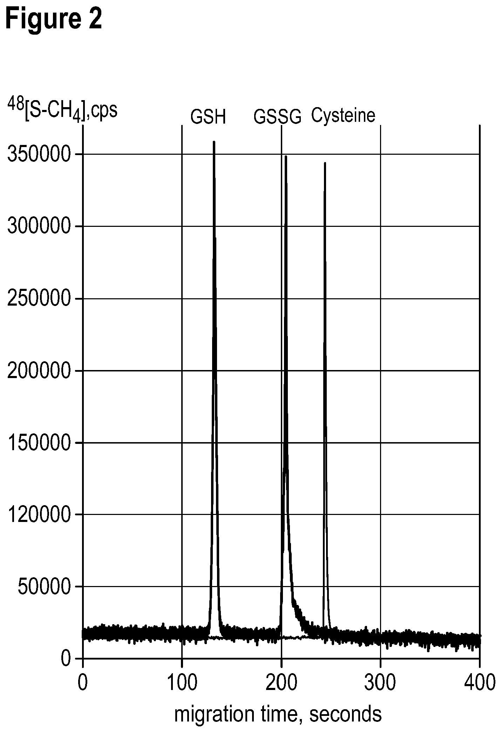

**Fig. 2****:** Sulfur speciation of GSH and GSSG standard compounds.

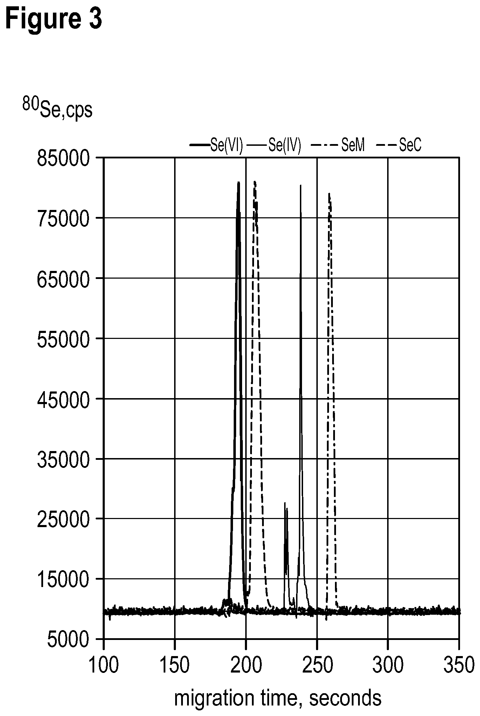

**Fig. 3**: Selenium speciation of Se(IV), Se(VI), selenomethionine (SeM) and selenocystine (SeC) standard compounds.

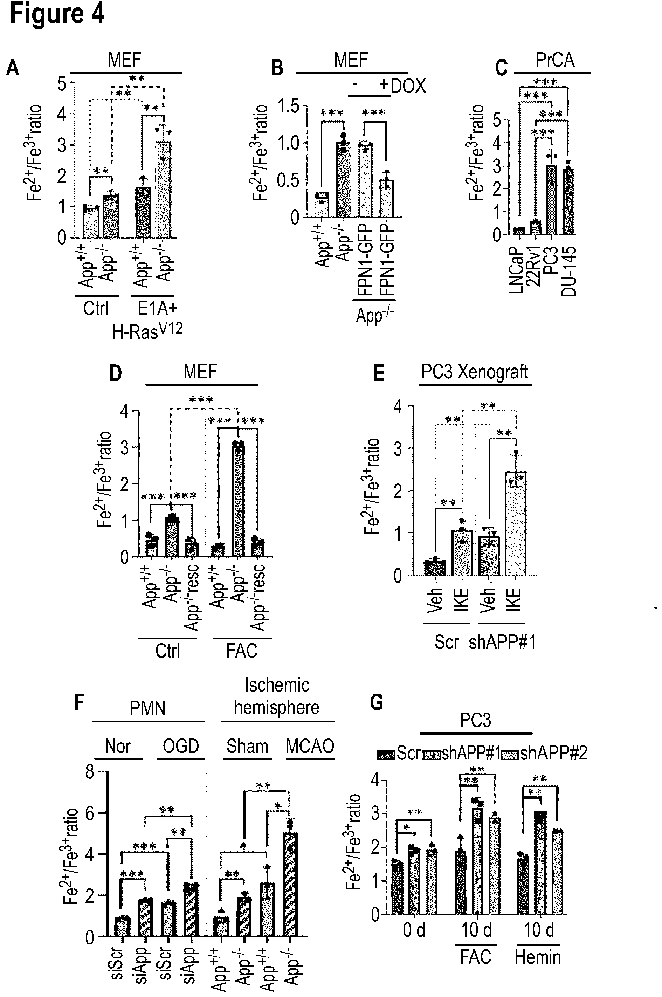

**Fig. 4****: Example for the application of the inventive method in various in vitro and in vivo models:** Fig. 4A shows early passages of SV40-immortalized App+/+ and App-/- MEFs and E1A/Ha-RasV12-transformed murine embryonal fibroblasts (MEF) variants. Respective lysates were analyzed via CE-ICP-MS analysis presented as absolute content of Fe²⁺ and Fe³⁺ in cell lysates of conditions in (A). Data are presented as Fe²⁺/Fe³⁺ ratio (n = 3, mean ± SD). Fig.B App-/- MEF cells stably expressing Dox-induced FPN-GFP vector (App-/- FPN-GFP). MEF cells were either left untreated (-Dox), incubated with either Dox (xx ng/ml) to force membrane-bound FPN1 re-expression Untreated cells served as untreated controls, while untransfected App-/- and App+/+ MEF lysates served as additional controls. CE-ICP-MS analysis showing absolute content of Fe²⁺ and Fe³⁺ in cell lysates of conditions. Fig. 4C Human PrCa cell models (LNCaP, 22Rc1, DU-145, PC3). CE-ICP-MS analysis showing absolute content of Fe²⁺ and Fe³⁺ in cell lysates of conditions. Data are presented as Fe²⁺/Fe³⁺ ratio (n = 3, mean ± SD). Fig. 4D CE-ICP-MS analysis showing absolute content of Fe²⁺ and Fe³⁺ in cell lysates of App+/+, App-/- and doxycycline (DOX)-induced re-constitution human APP751-isoform in App-/- (APP-/-resc MEF) murine embryonal fibroblasts (MEF) following 24-h treatment with 100 µM ferric ammonium citrate (FAC, 200 µM) or left untreated. Fig. 4E The human PrCa xenograft model was constructed by subcutaneously injecting the indicated PC3 cells in 5 mice. Twenty days after injection, mice were intraperitoneally treated with imidazole keton erastin (IKE, 50 mg/kg body weight) or vehicle (Veh) for 10 days and tumor growth was further monitored until day 40 (n = 10, each group). The tumor growth was measured by electronic caliper daily, calculated using the formula volume = 0.5 × length × width2 and are plotted as mean ± SD. Fe²⁺/Fe³⁺ ratio from lysates of PC3 xenografts analyzed by CE-ICP-MS.Fig. 4F PMN treated with scramble siRNA (siScr) or App-siRNA (siApp) under normoxia (Nor) and OGD condition (left panel). CE-ICP-MS analysis showing absolute content of Fe²⁺ and Fe³⁺ of PMN treated with scramble siRNA (siScr) or App-siRNA (siApp) under normoxia (Nor) and OGD condition or App+/+ and App-/- mice with or without MCAO surgery. Fig 4G FAC (200 µM) treatment for 5 and 10 days in PC3-scr and APPshRNA#1 and #2 cells. Fe²⁺/Fe³⁺ ratio in analyzed in cell lysates analogous to (B) analyzed by CE-ICP-MS (n = 3). Values are presented as mean ± SD

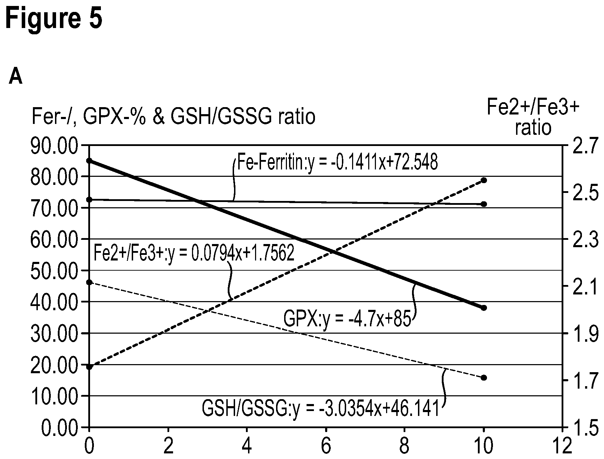

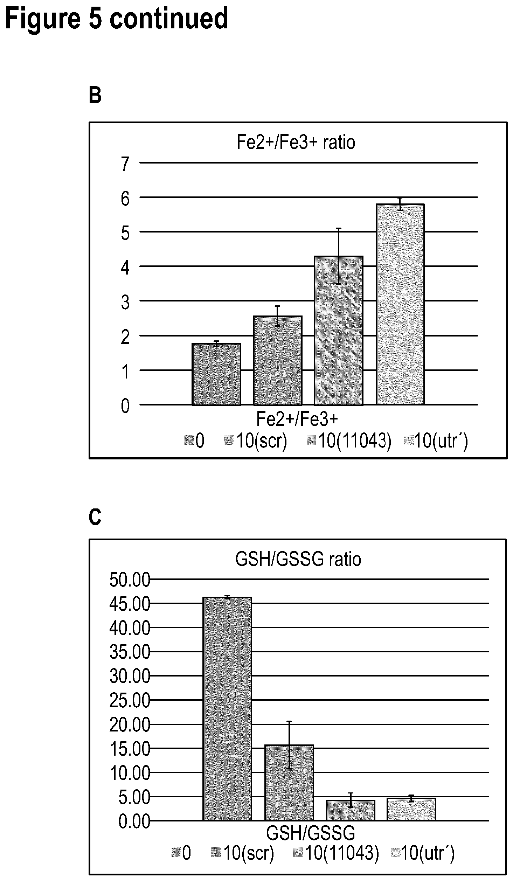

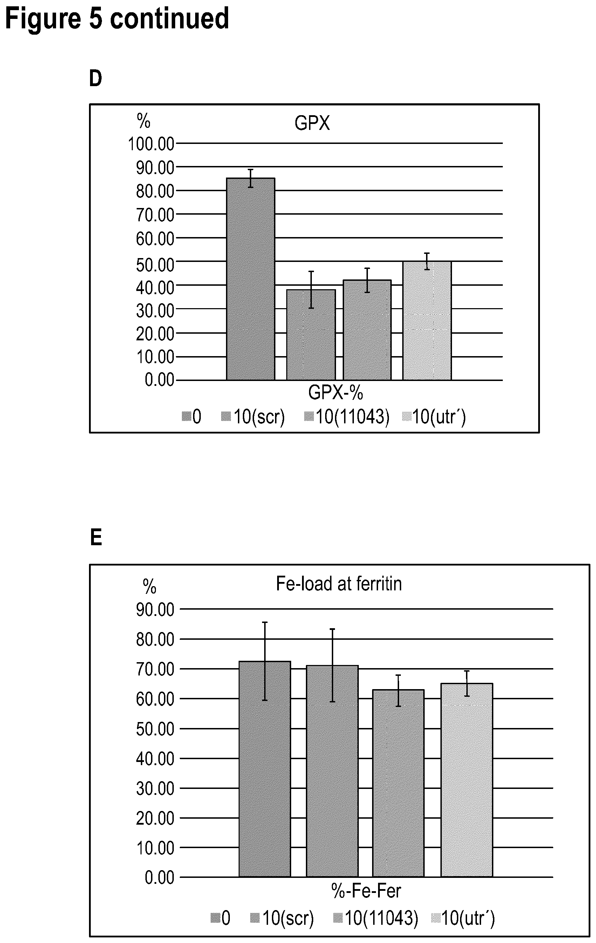

**Fig. 5****: Example for the application of the inventive method on the progressive development of prostate cancer cells:** Fig. 5A shows that with increasing development time: 1. The Fe²⁺/Fe³⁺ ratio increases due to increase of ROS/oxidative stress (OS). 2. The GSH/GSSG ratio decreases due to depletion of GSH as defense against oxidative stress. 3. GPX decreases due to exhaustion of GSH recovery mechanism 4. The Fe-load of ferritine is not affected. Figures 5B to 5E show the changes of certain markers in prostate cancer cells over 10 days in the presence of ROS promoters (#11043, #utr') or absence of ROS promoters (scr). Figure 5B: Fe2+/Fe3+ Ratio as signature for oxidative stress (OS). Figure 5C: GSH/GSSG ratio decreases due to depletion of GSH as defense against oxidative stress; Figure 5D: Relative Quantification of the GPX4 share of the selenium compounds. GPX4 decreases due to exhaustion of GSH recovery mechanism. Figure 5E: Fe-load of total Fe of ferritine. The Fe-load of ferritine is not affected.

### DETAILED DESCRIPTION OF THE INVENTION

The solution of the present invention is described in the following, exemplified in the appended examples, illustrated in the Figures and reflected in the claims.

### Definitions

It is noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

For example, less than 20 means less than the number indicated. Similarly, more than or greater than means more than or greater than the indicated number, f.e. more than 80 % means more than or greater than the indicated number of 80 %.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". When used herein "consisting of" excludes any element, step, or ingredient not specified.

DRC is dynamic reaction cell technology. The dynamic reaction cell or collision reaction cell, is a chamber placed before the traditional quadrupole chamber of an ICP-MS device, for eliminating isobaric interferences. See for example Yip, Y.; Sham, W (2007). "Applications of collision/reaction-cell technology in isotope dilution mass spectrometry". TrAC Trends in Analytical Chemistry. 26: 727. doi:10.1016/j.trac.2007.03.007*.*

sf-MS is a sector field mass spectrometer, as known to the person skilled in the art.

Qqq-MS is a triple quadrupole mass spectrometer, as known to the person skilled in the art.

The invention is related to an ex vivo analytic method for analysis of a sample (S1).

.The sample (S1) may comprise any fluid. The sample (S1) may comprise material obtained from a subject, preferably a human subject. Preferably the sample (S1) comprises a biofluid. More preferably, the biofluid is selected from the group consisting of a cell lysate, a tissue lysate, cerebrospinal fluid (CSF), serum, and urine. Preferably, samples are stored under inert gas immediately after collection or extract preparation at 0 to -100 °C, more preferably at -20 to -90 °C, most preferred at -75 to -80° °C, until analysis. Preferably, the inert gas is selected from the group consisting of nitrogen, and argon. Preferably, serum sample collection, in particular CSF is performed according to standard guidelines, such as e.g. Stanford Lumbar Puncture Guidelines (see: https://dx.stanford.edu/procedures/Procedures_LumbarPuncture.pdf)

In one embodiment, respective cell material is lysed into a buffer with gentle agitation, preferably cooled on ice. Preferably, the buffer is lysed for 10 to 60 minutes, more preferably for 20 to 50 minutes, most preferably 30 minutes. Preferably, the cell lysates were centrifuged at 5000 to 20000 g, more preferably at 8000 to 15000 g, most preferably at 10000 g. Preferably, the buffer contains:
i) PBS (phosphate buffered saline: for example comprising

| | |
|---|---|
| NaCl | 1,37 M |
| KCI | 27 mM |
| Na₂HPO₄ | 100 mM |
| KH₂PO₄ | 20 mM |
| pH | 7,2- |
| 7,6); | |

and/or
ii) sodium deoxycholate;
preferably in a concentration of 0.1 to 1 wt.-%, more preferably 0.3 to 7wt.-%, most preferably 0.5 wt.-% based on the overall mass of the sample (S1);
and/or
iii) NP-40 (Nonoxynol-40, CAS: 9016-45-9);
preferably in a concentration of 0.1 to 2% wt.-%, more preferably 0.2 to 1.5 wt.-%, most preferably 1% wt.-% based on the overall mass of the sample (S1).

Preferably, the protein concentration of the cell lysates is determined. Preferably, the protein concentration in the final sample (S1) is diluted, preferably with buffer as described above to a protein concentration of 0.1 to 5, more preferably to 0.5 to 3, most preferred 1 µg protein/µL (proteins as a result of the lysis of cells) in the sample (S1). Preferably, serum is diluted 1:10 to 1:100, more preferably 1: 60 to 1:90, most preferred 1:80. Preferably, serum or urine are diluted 1:1 to 1:4, more preferably 1:1.2 to 1:3, most preferably 1:1.5.

Preferably, the volume of sample (S1) is not more than 20 µl, more preferably not more than 15 µl, most preferably not more than 10 µl. Preferably, sample (S1) is the basis for analysis in step A) and B). Preferably, in step A) a part of sample (S1) is used and another part of sample (S1) is used in step B).

The ex vivo analytic method comprises the steps:
A) Determining Fe²⁺, Fe³⁺, Fe-Ferritin and total Fe content in sample (S1) and
B) Determining S and Se containing compounds content of sample (S1).

Step A) comprises the steps:
A1) Separating simultaneously Fe²⁺, Fe³⁺ and Fe-Ferritin present in a sample (S1); and
A2) Quantifying simultaneously Fe²⁺, Fe³⁺, Fe-Ferritin separated in step A1) and total Fe based on sample (S1).

"Fe-Ferritin" means in the context of the present invention, the amount of iron or iron ions bound to the iron storage protein Ferritin.

Total Fe content means the overall amount of iron present in the sample independent from its form, such as solved or bound to proteins etc..

Step A1) and/or A2) are preferably carried out within 1 to 15 minutes, more preferably within 3 to 10 minutes, most preferably 4 to 7 minutes, particular preferred within 5 minutes.

Seperating in step A1) is carried out with capillary electrophoresis.

Any known capillary electrophoresis device may be used. A capillary electrophoresis device comprises at least a capillary and a vial with a buffer solution at the inlet of the capillary and a vial with a buffer solution at the outlet of the capillary. If the term "inlet" is used, that means at the inlet of the capillary and the term "outlet", at the outlet of the capillary.

Seperation in step A1) using capillary electrophoresis may comprise a capillary preparation step before the actual separation. Capillary preparation includes purging and fill with leading electrolyte and stacking, preferably with a solution (E1) comprising HCl and MeOH; wherein optionally
i) the concentration of HCl is 5 to 15 weight-%, preferably 8 to 12 weight-%, more preferably 10 weight-%; based on the overall amount of the solution and/or
ii) the concentration of MeOH is 5 to 20 weight-%, preferably 7 to 15 weight-%, more preferably 10 weight-% based on the overall amount of the solution.

Preferably, purging is carried out at a pressure of 2 to 8 bar, more preferably 3 to 7 bar, most preferably 4 bar and/or for 0.5 to 5 min, more preferably 0.6 to 3 min, most preferably 1 min.

Preferably, the sample (S1) is injected into the capillary in step A1) at the inlet, more preferably after the capillary preparation step. Preferably, the injected volume of sample (S1) is not more than 5 to 30 nL, more preferably not more than 10 to 20 nL, most preferred not more than 15 nL. Preferably, the sample (S1) is injected wth a pressure of 100 to 600 mbar, more preferably 200 to 500 mbar, most preferably 300 mbar.

After injection of the sample, the capillary may be treated with an aqueous HCl solution (E2), preferably with a concentration of 0.005 to 0.2 mM, more preferably 0.01 to 0.1 mM, most preferably 0.05 mM and/or at a pressure of 100 to 600 mbar, more preferably 200 to 500 mbar, most preferably 300 mbar.

The electrolyte in step A1) at the inlet may be an aqueous HCl solution (E3), preferably with a concentration of 20 to 80 mM, more preferably 30 to 70 mM, most preferably 50 mM.

Preferably, the electrolyte at the inlet has a pressure of 200 to 800 mbar, more preferably 300 to 700 mbar, most preferably 600 mbar. Preferably at at voltage of 10 to 60 kV, more preferably 20 to 50 kV, most preferably 30 kV.

The electrolyte in step A1) at the outlet (E4) may be an aqueous HCl solution, preferably with a concentration of 1 to 15 mM, more preferably 3 to 8 mM, most preferably 5 mM.

The quantifying in step A2) is carried out with mass spectroscopy. The mass spectroscopy may comprise inductively coupled plasma mass spectroscopy (ICP-MS), preferably selected from the group consisting of ICP-DRC-MS, ICP-qqq-MS and ICP-sf-MS, more preferably ICP-DRC-MS; wherein optionally the DRC gas is NH₃. Preferably, argon is used as plasma gas in the mass spectroscopy and/or as nebulizer gas. Preferably, the measured isotope is ⁵⁶Fe.

The volume of sample (S1) used in step A1) and A2) combined may be not more than 30 nL, preferably not more than 20 nL, most preferably not more than 15 nL.

Step B) comprises the steps:
B1) Separating simultaneously S and Se containing compounds present in sample (S1);
   and
B2) Quantifying simultaneously S and Se containing compounds separated in step B1) of sample (S1).

Step B1) and/or B2) are preferably carried out within 1 to 15 minutes, more preferably within 3 to 10 minutes, most preferably 4 to 7 minutes, particular preferred within 5 minutes.

The one or more S (sulfur) containing compounds is selected from the group consisting of oxidized glutathione (GSSG), reduced glutathione (GSH), cystine, and/or cysteine, preferably oxidized glutathione (GSSG), reduced glutathione (GSH), and/or cysteine.

The one or more Se (selenium) containing compounds are selected from the group consisting of Se⁴⁺, Se⁶⁺, Se-methionine, Se-cystine, Se-cysteine, GPX4,Selenoprotein P, and/or Seleno sugars, preferably Se⁴⁺, Se⁶⁺, Se-methionine, Se-cysteine, Se-cystine and/or GPX4, more preferably Se⁴⁺, Se⁶⁺, Se-methionine, Se-cysteine and/or GPX4.

In the context of this invention, the term "Se-methionine" means selenomethionine, for example CAS number: 3211-76-5. In the context of this invention, the term "Se-cysteine" means selenocysteine, for example CAS number: 10236-58-5. GPX4 is Glutathione peroxidase 4, a selenium containing enzyme. "Se-cystine" means selenocystine, for example CAS number: 29621-88-3.

Seperating in step B1) is carried out with capillary electrophoresis.

Seperation in step B1) using capillary electrophoresis may comprise a capillary preparation step before the actual separation. Capillary preparation includes purging and filling with leading electrolyte (E5) and stacking, preferably with a solution comprising a borate buffer, and CTAB Cetyltrimethylammonium bromide), wherein optionally
i) the concentration of borate is 50 to 200 mM, preferably 80 to 150 mM, more preferably 90 mM; and/or
ii) the borate is sodium borate or potassium borate, preferably sodium borate;
iii) the concentration of CTAB is 1 to 10 mM, preferably 1 to 5 mM, more preferably 3 mM.

Preferably, purging and filling with leading electrolyte and stacking in step B1) is carried out at 2 to 8 bar, more preferably 3 to 7 bar, most preferably 4 bar and/or for 0.5 to 5 min, more preferably 0.6 to 3 min, most preferably 1 min.

Preferably, the sample (S1) is injected into the capillary in step B1), more preferably after the capillary preparation step. Preferably, the injected volume of sample (S1) in step B1) is not more than 5 to 30 nL, more preferably not more than 10 to 20 nL, most preferred not more than 15 nL. Preferably, the sample (S1) is injected wth a pressure of 100 to 600 mbar, more preferably 200 to 500 mbar, most preferably 300 mbar.

The electrolyte (E6) in step B1) at the inlet may be an aqueous solution comprising HCl , preferably in concentration of 5 to 100 mM, more preferably 10 to 50 mM, most preferably 20 mM.

The electrolyte (E7) in step B1) at the inlet may be an aqueous solution comprising TMAH (Tetramethylammoniumhydroxide), EtOH, and/or CTAB (Cetyltrimethylammonium bromide) , wherein optionally
i) the concentration of TMAH is 0.5 to 10 weight-%, preferably 1 to 5 weight-%, more preferably 3 weight-%; based on the overall amount of the aqueous solution and/or
ii) the concentration of EtOH is 5 to 20 weight-%, preferably 7 to 15 weight-%, more preferably 10 weight-%; based on the overall amount of the aqueous solution and/or
iii) the concentration of CTAB is 1 to 10 mM, preferably 1 to 5 mM, more preferably 3 mM.

Preferably, the electrolyte at the inlet has a pressure of 200 to 800 mbar, more preferably 300 to 700 mbar, most preferably 500 mbar. Preferably at voltage of 10 to 60 kV, more preferably 20 to 50 kV, most preferably 30 kV.

The electrolyte in step B1) at the outlet may be an aqueous solution (E8) comprising NH₄-acetate preferably in concentration of 1 to 15 mM, more preferably 3 to 8 mM, most preferably 5 mM.

Preferably, in step B1), the sample is at the inlet.

The quantifying in step B2) is carried out with mass spectroscopy. The mass spectroscopy may comprise inductively coupled plasma (ICP) mass spectroscopy, preferably selected from the group consisting of ICP-DRC-MS, ICP-qqq-MS and ICP-sf-MS, more preferably ICP-DRC-MS; wherein optionally the DRC gas is CH₄. Preferably, the use of CH₄ results in a mass shift of the sulfur species from m/z = 32 to m/z = 48. Preferably, sulfur compounds are detected as m/z = 48 peak. Preferably, argon is used as plasma gas in the mass spectroscopy and/or as nebulizer gas. Preferably, the measured isotopes are ⁴⁸[S-CH₄], ⁷⁸Se, and/or ⁸⁰Se.

The volume of sample (S1) used in step B1) and B2) combined is not more than 30 nL, preferably not more than 20 nL, most preferably not more than 15 nL.

In one embodiment, ex vivo analytic method for analysis of a sample (S1), comprising the steps:
A) Determining Fe²⁺, Fe³⁺, Fe-Ferritin and total Fe content in sample (S1), comprising the steps:
   A1) Separating simultaneously Fe²⁺, Fe³⁺ and Fe-Ferritin present in a sample (S1) with capillary electrophoresis; wherein the electrolyte (E3) at the inlet is an aqueous solution comprising HCl, wherein
      the HCl solution (E3), has a concentration of 20 to 80 mM, preferably 30 to 70 mM, more preferably 50 mM.
   A2) Quantifying simultaneously Fe²⁺, Fe³⁺, Fe-Ferritin separated in step A1) and total Fe based on sample (S1) with ICP-DRC-MS;
B) Determining S and Se containing compounds content of sample (S1), comprising the steps:
   B1) Separating simultaneously S and Se containing compounds present in sample (S1) with capillary electrophoresis;
   B2) Quantifying simultaneously S and Se containing compounds separated in step B1) of sample (S1) with ICP-DRC-MS, wherein the DRC gas is CH₄.

In a further embodiment, ex vivo analytic method for analysis of a sample (S1), comprising the steps:
A) Determining Fe²⁺, Fe³⁺, Fe-Ferritin and total Fe content in sample (S1), comprising the steps:
   A1)
      I) Capillary preparation, including purging and filling with leading electrolyte (E1) and stacking, with a solution comprising HCl and MeOH; wherein
         i) the concentration of HCl is 5 to 15 weight-%, preferably 8 to 12 weight-%, more preferably 10 weight-%; based on the overall amount of the solution and
         ii) the concentration of MeOH is 5 to 20 weight-%, preferably 7 to 15 weight-%, more preferably 10 weight-% based on the overall amount of the solution;
         purging is carried out at a pressure of 2 to 8 bar, more preferably 3 to 7 bar, most preferably 4 bar;
      II)Separating simultaneously Fe²⁺, Fe³⁺ and Fe-Ferritin present in a sample (S1) with capillary electrophoresis; wherein separating comprises
         i) injection of a part of sample (S1);
         ii) wherein the electrolyte at the outlet (E4) is an aqueous HCl solution, with a concentration of 1 to 15 mM, preferably 3 to 8 mM, more preferably 5 mM;and
         iii) wherein the electrolyte (E3) at the inlet is an aqueous solution comprising HCl, wherein the concentration of HCl is 20 to 100 mM preferably 30 to 80 mM, more preferably 50 mM, based on the overall amount of the aqueous solution;
            A2) Quantifying simultaneously Fe²⁺, Fe³⁺, Fe-Ferritin separated in step A1) and total Fe based on sample (S1) with ICP-DRC-MS;
B) Determining S and Se containing compounds content of sample (S1), comprising the steps:
   B1)
      I) Capillary preparation including purging and filling with leading electrolyte (E5) and stacking, with an aqueous solution (E5) comprising a borate buffer and CTAB, wherein the concentration of borate is 50 to 200 mM, preferably 80 to 150 mM, more preferably 100 mM.
      II) Separating simultaneously S and Se containing compounds present in sample (S1) with capillary electrophoresis;
         i) injection of a part of sample (S1);
         ii) the electrolyte (E7) at the inlet is an aqueous solution comprising TMAH , EtOH and CTAB (Cetyltrimethylammonium bromide), wherein the concentration of TMAH is 1 to 10 mM,, preferably 2 to 5 mM, more preferably 3 mM,; the concentration of TMAH is 0.5 to 10 weight-%, preferably 1 to 5 weight-%, more preferably 3 and weight-%; and/or the concentration of EtOH is 5 to 20 weight-%, preferably 7 to 15 weight-%, more preferably 10 weight-% based on the overall amount of the aqueous solution.
   B2) Quantifying simultaneously S and Se containing compounds separated in step B1) of sample (S1) with ICP-DRC-MS, wherein the DRC gas is CH₄.

The analytic method may further comprise providing information on the results of the analytic method, preferably to a subject.

The invention further comprises the use of the analytic method for an ex vivo diagnosis of oxidative stress, ferroptosis, and ferroptosis-associated diseases. Preferably, the ferroptosis-associated disease is hemochromatosis, or sideroblastic anemia.

The invention further comprises the use of the analytic method for an ex vivo diagnosis of prostate cancer, melanoma, diabetes multiple sclerosis, and myelodysplastic syndrome, preferably prostate cancer.

The invention further comprises a kit for performing the analytic method, as described above, comprising
i) devices for performing the analytic method; and
ii) a HCl solution, preferably with a concentration of 20 to 80 mM, more preferably 30 to 70 mM, most preferably 50 mM; for performing step A1) and
iii) a HCl solution, preferably with a concentration of 1 to 15 mM, more preferably 3 to 8 mM, most preferably 5 mM; for performing step A1) and
iv) CH₄ gas for performing step B2).

### EXAMPLES OF THE INVENTION

### Capillary zone electrophoresis (CZE)

A "PrinCe 706" CE system from PrinCe Technologies B.V. (Emmen, The Netherlands) is employed. Temperature settings for sample/buffer tray and capillary are set at 20 oC by air cooling, each. An uncoated capillary with dimension 100 cm x 50 µm ID (CS-Chromatographie Service GmbH, Langerwehe, Germany) was used for separation and hyphenation to the ICP - MS. A CE-ICP-MS interface was installed which provided the electrical connection between CE capillary end and outlet electrode. The self-aspiration mode allowed for best flow rate adjustment and reduced (avoided) suction flow.

**Table 1** and **2** show the consecutive steps of the analysis:
First shot for speciation of Fe-ferritin, Fe²⁺ and Fe³⁺.
Section A: capillary preparation steps before measurement;
Section B: electrolytes and conditions during analysis.
Section C: Detection: ICP-DRC-MS conditions during analysis

**Table 1: First shot**

| **A** | **Capillary preparation** | | |
|---|---|---|---|
| | Purge and fill with leading electrolyte and stacking | 10% HCl/ 10% MeOH | 4 bar, 1 min |
| | Injection | | 300 mbar, 6 s |
| | Stacking with terminating electrolyte | 0.05mM HCl | 300 mbar, 6 s |

| **B** | **Analysis by CZE separation** | | |
|---|---|---|---|
| | Inlet electrolyte | 50 mM HCl | 600 mbar, 5 min, + 30 kV |
| | Outlet/sheath electrolyte at ICP-MS interface | 5 mM HCl | Interface working with self-aspiration ca. 100 µL/min |

| **C** | **ICP-DRC-MS detection** | | |
|---|---|---|---|
| | Conditions | RF power | 1,250W |
| | | Plasma gas | 16 L Ar/min |
| | | Nebulizer gas | 0.94 L Ar/min. |
| | | Dwell time | 50 ms |
| | | DRC gas | NH₃ |
| | | DRC gas flow | 0.58 mL/min |
| | | DRC rejection parameter | 0.58 |
| | | | ⁵⁶Fe |
| | | Measured isotope | |

Second shot for speciation of GSH, GSSG, cysteine, and GPX4 and other seleno species
Section A: capillary preparation steps before measurement;
Section B: electrolytes and conditions during analysis.
Section C: Detection: ICP-DRC-MS conditions during analysis

**Table 2: Second Shot**

| **A** | **Capillary preparation** | | |
|---|---|---|---|
| | Purge and fill with background electrolyte | 100 mM borate buffer, 3mM CTAB, pH 7.8 | 4 bar, 1 min |
| | Injection | | 300 mbar, 10 s |

| **B** | **Analysis** | | |
|---|---|---|---|
| | Inlet electrolyte | 3% TMAH, 10% EtOH, 3 mM CTAB | 500 mbar, 5 min, + 30 kV |
| | Outlet/sheath electrolyte at ICP-MS interface | 5 mM NH₄-acetate | Interface working with self-aspiration ca. 100 µL/min |

| | **ICP-DRC-MS detection** | | |
|---|---|---|---|
| | Conditions | RF power | 1,250W |
| | | Plasma gas | 16 L Ar/min |
| | | Nebulizer gas | 0.94 LAr/min. |
| | | Dwell time | 50 ms |
| | | DRC gas | CH₄ |
| | | DRC gas flow | 0.6 mL/min |
| | | DRC rejection parameter | 0.60 |
| | | | ⁴⁸[S-CH₄], ⁷⁸Se, ⁸⁰Se |
| | | Measured isotopes | |

### Inductively coupled plasma mass spectrometry (ICP-MS) as CE detector

A Nexlon 300 D (Perkin Elmer, Sciex, Toronto, Canada) was operated as ICP-MS system for the on-line detection of CE-efflux. The RF power was set to 1250 W, the plasma gas was 16 LAr/min. The nebulizer gas was optimized and finally set to 0.98 LAr/min.
During the first analysis shot (iron speciation) the isotope ⁵⁶Fe was measured in dynamic reaction cell (DRC) mode. The dwell time was 50 ms. Ammonia was used as DRC gas (0.58 ml NH3/min) and DRC rejection parameter was set to 0.58.

During the second analysis shot (sulfur and selenium speciation) the isotopes ⁴⁸[S-CH₄], ⁷⁸Se, ⁸⁰Se were measured in dynamic reaction cell (DRC) mode. The dwell time was 50 ms. Methane was used as DRC gas (0.60 ml CH₄/min) and DRC rejection parameter was set to 0.60.

### REFERENCES

1. Galluzzi, L., et al., Molecular mechanisms of cell death: recommendations of the Nomenclature Committee on Cell Death 2018. Cell Death & Differentiation, 2018. 25(3): p. 486-541.
2. Ashraf, A., M. Clark and P. W. So (2018). "The Aging of Iron Man." Frontiers in Aging Neuroscience 10.
3. Hare, D. J., M. Arora, N. L. Jenkins, D. I. Finkelstein, P. A. Doble and A. I. Bush (2015). "Is early-life iron exposure critical in neurodegeneration?" Nat Rev Neurol 11(9): 536-544.
4. Dixon, S.J. and B.R. Stockwell, The role of iron and reactive oxygen species in cell death. Nature chemical biology, 2014. 10(1): p. 9-17.
5. Yang, W.S., et al., Regulation of ferroptotic cancer cell death by GPX4. Cell, 2014. 156(1-2): p. 317-31.
6. Stockwell, B.R., et al., Ferroptosis: A Regulated Cell Death Nexus Linking Metabolism, Redox Biology, and Disease. Cell, 2017. 171(2): p. 273-285.
7. Dixon, S.J., et al., Pharmacological inhibition of cystine-glutamate exchange induces endoplasmic reticulum stress and ferroptosis. eLife, 2014. 3: p. e02523.
8. Dixon, S.J. and B.R. Stockwell, The role of iron and reactive oxygen species in cell death. Nature chemical biology, 2014. 10(1): p. 9-17.
9. Michalke, B. et al., Front Chem., 2019.7: p.136.
10.
   a) Dixon, S.J., et al., Ferroptosis: an iron-dependent form of nonapoptotic cell death. Cell, 2012. 149(5): p. 1060-72.
   b) Dixon, S.J., et al., Pharmacological inhibition of cystine-glutamate exchange induces endoplasmic reticulum stress and ferroptosis. eLife, 2014. 3: p. e02523.
   c) Dixon, S.J. and B.R. Stockwell, The role of iron and reactive oxygen species in cell death. Nature chemical biology, 2014. 10(1): p. 9-17.

## Claims

1. An ex vivo analytic method for analysis of a sample (S1), comprising the steps:
A) Determining Fe²⁺, Fe³⁺, Fe-Ferritin and total Fe content in sample (S1), comprising the steps:
A1) Separating simultaneously Fe²⁺, Fe³⁺ and Fe-Ferritin present in a sample (S1) with capillary electrophoresis;
A2) Quantifying simultaneously Fe²⁺, Fe³⁺, Fe-Ferritin separated in step A1) and total Fe based on sample (S1) with mass spectroscopy;
B) Determining S and Se containing compounds content of sample (S1), comprising the steps
B1) Separating simultaneously S and Se containing compounds present in sample (S1) with capillary electrophoresis;
B2) Quantifying simultaneously S and Se containing compounds separated in step B1) of sample (S1) with mass spectroscopy;
wherein the S containing compounds are selected from the group consisting of oxidized glutathione (GSSG), reduced glutathione (GSH), cystine, and/or cysteine;
and the Se containing compounds are selected from Se⁴⁺, Se⁶⁺, Se-methionine, Se-cysteine GPX4, Selenoprotein P, and/or Seleno sugars.

2. The analytic method of claim 1, wherein
I) in step A1) the sample is injected at the inlet;
II) after injection of the sample, the capillary is treated with an electrolyte (E2) in step A1), wherein the electrolyte (E2) is an aqueous HCl solution, preferably with a concentration of 0.005 to 0.2 mM, more preferably 0.01 to 0.1 mM, most preferably 0.05 mM; and/or
III) wherein seperation in step A1) using capillary electrophoresis comprises a capillary preparation step before the actual separation, including purging and filling with leading electrolyte and stacking, preferably with a solution (E1) comprising HCl and MeOH; wherein optionally
i) the concentration of HCl is 5 to 15 weight-%, preferably 8 to 12 weight-%, more preferably 10 weight-%; based on the overall amount of the solution and/or
ii) the concentration of MeOH is 5 to 20 weight-%, preferably 7 to 15 weight-%, more preferably 10 weight-% based on the overall amount of the solution and/or
IV) the electrolyte (E3) in step A1) at the inlet is an aqueous HCl solution, preferably with a concentration of 20 to 80 mM, more preferably 30 to 70 mM, most preferably 50 mM; and/or
V) the electrolyte (E4) in step A1) at the outlet is an aqueous HCl solution, preferably with a concentration of 1 to 15 mM, more preferably 3 to 8 mM, most preferably 5 mM; and/or
VI) wherein separation in step B1) using capillary electrophoresis comprises a capillary preparation step before the actual separation, including purging and filling with leading electrolyte (E5) and stacking, preferably with a solution comprising a borate buffer, and CTAB Cetyltrimethylammonium bromide), wherein optionally
i) the concentration of borate is 50 to 200 mM, preferably 80 to 150 mM, more preferably 90 mM; and/or
ii) the concentration of CTAB is 1 to 10 mM, preferably 1 to 5 mM, more preferably 3 mM; and/or
VII) the electrolyte in step B1) at the inlet is an aqueous solution (E6) comprising HCl, preferably in concentration of 5 to 100 mM, more preferably 10 to 50 mM, most preferably 20 mM; and/or
VIII) in step B1) the sample is at the inlet; and/or
IX) the electrolyte (E7) in step B1) at the inlet is an aqueous solution comprising TMAH (Tetramethylammoniumhydroxide), EtOH, and/or CTAB (Cetyltrimethylammonium bromide, wherein optionally
i) the concentration of TMAH is 0.5 to 10 weight-%, preferably 1 to 5 weight-%, more preferably 3 weight-%; based on the overall amount of the aqueous solution and/or
ii) the concentration of EtOH is 5 to 20 weight-%, preferably 7 to 15 weight-%, more preferably 10 weight-%; based on the overall amount of the aqueous solution and/or
iii) the concentration of CTAB is 1 to 10 mM, preferably 1 to 5 mM, more preferably 3 mM; and/or
X) the electrolyte (E8) in step B2) at the outlet is an aqueous solution comprising NH₄-acetate preferably in concentration of 1 to 15 mM, more preferably 3 to 8 mM, most preferably 5 mM.

3. The analytic method of claim 1 or 2, wherein in step A2) and/or B2) quantifying is carried out with application of inductively coupled plasma mass spectroscopy (ICP), wherein optionally the inductively coupled plasma mass spectroscopy (ICP) is
I) selected from the group consisting of ICP-DRC-MS, ICP-qqq-MS and ICP-sf-MS or
II) ICP-DRC-MS, wherein optionally
i) in step A2) the DRC gas is NH₃ and/or
ii) in step B2) the DRC gas is CH₄.

4. The analytic method of any one of claims 1 to 3, wherein the sample (S1) comprises material obtained from a subject, preferably a human subject.

5. The analytic method of any one of claims 1 to 4, wherein the sample (S1) comprises a biofluid, preferably selected from the group consisting of a cell lysate, a tissue lysate, CSF, serum, and urine.

6. The analytic method of any one of claims 1 to 5, wherein
I) the volume of sample (S1) is not more than 20 µl, preferably not more than 15 µl, more preferably not more than 10 µl; and/or
II) the volume used in step A1) and A2) combined is not more than 30 nL, preferably not more than 20 nL, most preferably not more than 15 nL; and/or
III) the volume used in step B1) and B2) combined is not more than 30 nL, preferably not more than 20 nL, most preferably not more than 15 nL.

7. The analytic method of any one of claims 1 to 6, wherein
I) step A1) and A2) and/or
II) step B1) and B2)
are carried out within 1 to 15 minutes, preferably within 3 to 10 minutes, more preferably 4 to 7 minutes, most preferably within 5 minutes.

8. The analytic method of any one of claims 1 to 7, wherein step A) and B) are carried out sequentially, preferably step A) before step B).

9. The analytic method of any one of claim 1 to 8, further comprising providing information on the results of the analytic method, preferably to a subject.

10. Use of the analytic method of any one of claims 1 to 9 for an ex vivo diagnosis of oxidative stress.

11. Use of the analytic method of any one of claims 1 to 9 for an ex vivo diagnosis of ferroptosis.

12. Use of the analytic method of any one of claims 1 to 9 for an ex vivo diagnosis of ferroptosis-associated diseases, preferably hemochromatosis, or sideroblastic anemia .

13. The use of the analytic method of any one of claims 1 to 9 for an ex vivo diagnosis of prostate cancer, melanoma, multiple sclerosis, diabetes and myelodysplastic syndrome.

14. A kit for performing the analytic method of any one of claims 1 to 9, comprising
i) devices for performing the analytic method using capillary electrophoresis coupled to mass spectrometry; and
ii) a HCl solution serving as electrolyte (E3) at the inlet, preferably with a concentration of 20 to 80 mM, more preferably 30 to 70 mM, most preferably 50 mM; for performing capillary electrophoresis (step A1) and
iii) a HCl solution serving as electrolyte (E4) at the outlet, preferably with a concentration of 1 to 15 mM, more preferably 3 to 8 mM, most preferably 5 mM; for performing capillary electrophoresis (step A1) and
iv) CH₄ gas for performing mass spectrometry (step B2).

## Patentansprüche

1. Ex-vivo-Analysemethode zur Analyse einer Probe (S1), umfassend die folgenden Schritte:
A) Bestimmung von Fe²⁺ , Fe³⁺ , Fe-Ferritin und des Gesamt-Fe-Gehalts in der Probe (S1), umfassend die folgenden Schritte:
A1) Gleichzeitige Trennung von Fe²⁺ , Fe³⁺ und Fe-Ferritin, die in einer Probe (S1) vorhanden sind, mittels Kapillarelektrophorese;
A2) gleichzeitige Quantifizierung von Fe²⁺ , Fe³⁺ , dem in Schritt A1) abgetrennten Fe-Ferritin und dem Gesamt-Fe-Gehalt der Probe (S1) mittels Massenspektroskopie;
B) Bestimmung des Gehalts an S- und Se-haltigen Verbindungen in der Probe (S1), umfassend die folgenden Schritte
B1) Gleichzeitige Trennung der in der Probe (S1) vorhandenen S- und Se-haltigen Verbindungen mittels Kapillarelektrophorese;
B2) Gleichzeitige Quantifizierung der in Schritt B1) aus der Probe (S1) getrennten S- und Se-haltigen Verbindungen mittels Massenspektroskopie;
wobei die S-haltigen Verbindungen aus der Gruppe ausgewählt sind, die aus oxidiertem Glutathion (GSSG), reduziertem Glutathion (GSH), Cystin und/oder Cystein besteht;
und die selenhaltigen Verbindungen aus Se⁴⁺ , Se⁶⁺ , Se-Methionin, Se-Cystein, GPX4, Selenoprotein P und/oder Selenozuckern ausgewählt sind.

2. Die Analysemethode nach Anspruch 1, wobei
I) in Schritt A1) die Probe am Einlass injiziert wird;
II) nach der Injektion der Probe die Kapillare in Schritt A1) mit einem Elektrolyten (E2) behandelt wird, wobei der Elektrolyt (E2) eine wässrige HCl-Lösung ist, vorzugsweise mit einer Konzentration von 0,005 bis 0,2 mM, noch bevorzugter 0,01 bis 0,1 mM, am meisten bevorzugt 0,05 mM; und/oder
III) wobei die Trennung in Schritt A1) mittels Kapillarelektrophorese einen Kapillaraufbereitungsschritt vor der eigentlichen Trennung umfasst, einschließlich Spülen und Befüllen mit einem Vorlaufelektrolyten sowie Stapeln, vorzugsweise mit einer Lösung (E1), die HCl und MeOH enthält; wobei optional
i) die Konzentration von HCl 5 bis 15 Gew.-%, vorzugsweise 8 bis 12 Gew.-%, noch bevorzugter 10 Gew.-% beträgt; bezogen auf die Gesamtmenge der Lösung und/oder
ii) die Konzentration von MeOH 5 bis 20 Gew.-%, vorzugsweise 7 bis 15 Gew.-%, am meisten bevorzugt 10 Gew.-% beträgt, bezogen auf die Gesamtmenge der Lösung und/oder
IV) der Elektrolyt (E3) in Schritt A1) am Einlass eine wässrige HCl-Lösung ist, vorzugsweise mit einer Konzentration von 20 bis 80 mM, noch bevorzugter 30 bis 70 mM, am meisten bevorzugt 50 mM; und/oder
V) der Elektrolyt (E4) in Schritt A1) am Auslass eine wässrige HCl-Lösung ist, vorzugsweise mit einer Konzentration von 1 bis 15 mM, noch bevorzugter 3 bis 8 mM, am meisten bevorzugt 5 mM; und/oder
VI) wobei die Trennung in Schritt B1) mittels Kapillarelektrophorese einen Kapillaraufbereitungsschritt vor der eigentlichen Trennung umfasst, einschließlich Spülen und Befüllen mit dem führenden Elektrolyten (E5) sowie dem Aufschichten von " ", vorzugsweise mit einer Lösung, die einen Boratpuffer und CTAB (Cetyltrimethylammoniumbromid) enthält, wobei optional
i) die Boratkonzentration 50 bis 200 mM, vorzugsweise 80 bis 150 mM, am meisten bevorzugt 90 mM beträgt; und/oder
ii) die Konzentration an CTAB 1 bis 10 mM, vorzugsweise 1 bis 5 mM, am bevorzugtesten 3 mM beträgt; und/oder
VII) Der Elektrolyt in Schritt B1) am Einlass ist eine wässrige Lösung (E6), die HCl enthält, vorzugsweise in einer Konzentration von 5 bis 100 mM, noch bevorzugter von 10 bis 50 mM, am meisten bevorzugt von 20 mM; und/oder
VIII) in Schritt B1) befindet sich die Probe am Einlass; und/oder
IX) der Elektrolyt (E7) in Schritt B1) am Einlass ist eine wässrige Lösung, die TMAH (Tetramethylammoniumhydroxid), EtOH und/oder CTAB (Cetyltrimethylammoniumbromid) enthält, wobei optional
i) die Konzentration von TMAH 0,5 bis 10 Gew.-%, vorzugsweise 1 bis 5 Gew.-%, am bevorzugtesten 3 Gew.-% beträgt; bezogen auf die Gesamtmenge der wässrigen Lösung und/oder
ii) die Konzentration von EtOH 5 bis 20 Gew.-%, vorzugsweise 7 bis 15 Gew.-%, noch bevorzugter 10 Gew.-% beträgt; bezogen auf die Gesamtmenge der wässrigen Lösung und/oder
iii) die Konzentration von CTAB 1 bis 10 mM, vorzugsweise 1 bis 5 mM, am meisten bevorzugt 3 mM beträgt; und/oder
X) der Elektrolyt (E8) in Schritt B2) am Auslass eine wässrige Lösung ist, die NH₄-Acetat enthält, vorzugsweise in einer Konzentration von 1 bis 15 mM, noch bevorzugter 3 bis 8 mM, am meisten bevorzugt 5 mM.

3. Analysemethode nach Anspruch 1 oder 2, wobei in Schritt A2) und/oder B2) die Quantifizierung unter Anwendung der induktiv gekoppelten Plasma-Massenspektroskopie (ICP) durchgeführt wird, wobei die induktiv gekoppelte Plasma-Massenspektroskopie (ICP) gegebenenfalls
I) aus der Gruppe ausgewählt ist, bestehend aus ICP-DRC-MS, ICP-qqq-MS und ICP-sf-MS, oder
II) ICP-DRC-MS ist, wobei optional
i) in Schritt A2) das DRC-Gas NH₃ ist und/oder
ii) in Schritt B2) das DRC-Gas CH₄ ist.

4. Das Analyseverfahren nach einem der Ansprüche 1 bis 3, wobei die Probe (S1) Material umfasst, das von einem Probanden, vorzugsweise einem menschlichen Probanden, gewonnen wurde.

5. Das Analyseverfahren nach einem der Ansprüche 1 bis 4, wobei die Probe (S1) eine Körperflüssigkeit umfasst, vorzugsweise ausgewählt aus der Gruppe bestehend aus einem Zelllysat, einem Gewebelysat, Liquor_{,} Serum und Urin.

6. Das Analyseverfahren nach einem der Ansprüche 1 bis 5, wobei
I) das Volumen der Probe (S1) nicht mehr als 20 µl, vorzugsweise nicht mehr als 15 µl, am bevorzugtesten nicht mehr als 10 µl beträgt; und/oder
II) das in den Schritten A1) und A2) insgesamt verwendete Volumen nicht mehr als 30 nL, vorzugsweise nicht mehr als 20 nL, am bevorzugtesten nicht mehr als 15 nL beträgt; und/oder
III) das in den Schritten B1) und B2) insgesamt verwendete Volumen nicht mehr als 30 nL, vorzugsweise nicht mehr als 20 nL, am bevorzugtesten nicht mehr als 15 nL beträgt.

7. Analysemethode nach einem der Ansprüche 1 bis 6, wobei
I) die Schritte A1) und A2) und/oder
II) die Schritte B1) und B2)
innerhalb von 1 bis 15 Minuten, vorzugsweise innerhalb von 3 bis 10 Minuten, noch bevorzugter innerhalb von 4 bis 7 Minuten, am meisten bevorzugt innerhalb von 5 Minuten durchgeführt werden.

8. Das Analyseverfahren nach einem der Ansprüche 1 bis 7, wobei die Schritte A) und B) nacheinander durchgeführt werden, vorzugsweise Schritt A) vor Schritt B).

9. Das Analyseverfahren nach einem der Ansprüche 1 bis 8, das ferner das Bereitstellen von Informationen über die Ergebnisse des Analyseverfahrens, vorzugsweise an eine Testperson, umfasst.

10. Verwendung des Analyseverfahrens nach einem der Ansprüche 1 bis 9 zur Ex-vivo-Diagnose von oxidativem Stress.

11. Verwendung des Analyseverfahrens nach einem der Ansprüche 1 bis 9 zur Ex-vivo-Diagnose von Ferroptose.

12. Verwendung des analytischen Verfahrens gemäß einem der Ansprüche 1 bis 9 zur Ex-vivo-Diagnose von mit Ferroptose assoziierten Erkrankungen, vorzugsweise Hämochromatose oder sideroblastischer Anämie.

13. Verwendung des Analyseverfahrens nach einem der Ansprüche 1 bis 9 zur Ex-vivo-Diagnose von Prostatakrebs, Melanom, Multipler Sklerose, Diabetes und myelodysplastischem Syndrom.

14. Ein Kit zur Durchführung des Analyseverfahrens nach einem der Ansprüche 1 bis 9, umfassend
i) Vorrichtungen zur Durchführung des Analyseverfahrens unter Verwendung von Kapillarelektrophorese in Verbindung mit Massenspektroskopie; und
ii) eine als Elektrolyt (E3) am Einlass dienende HCl-Lösung, vorzugsweise mit einer Konzentration von 20 bis 80 mM, noch bevorzugter 30 bis 70 mM, am meisten bevorzugt 50 mM; zur Durchführung der Kapillarelektrophorese (Schritt A1) und
iii) einer HCl-Lösung, die als Elektrolyt (E4) am Auslass dient, vorzugsweise mit einer Konzentration von 1 bis 15 mM, noch bevorzugter 3 bis 8 mM, am meisten bevorzugt 5 mM; zur Durchführung der Kapillarelektrophorese (Schritt A1) und
iv) CH₄-Gas zur Durchführung der Massenspektrometrie (Schritt B2).

## Revendications

1. Procédé d'analyse ex vivo destiné à analyser un échantillon (S1), comprenant les étapes suivantes :
A) Déterminer la teneur en Fe²⁺ , Fe³⁺ , Fe-ferritine et en fer total dans l'échantillon (S1), comprenant les étapes suivantes :
A1) Séparer simultanément le Fe²⁺ , le Fe³⁺ et la ferritine de fer présents dans un échantillon (S1) par électrophorèse capillaire ;
A2) Quantifier simultanément le Fe²⁺ , le Fe³⁺ , la ferritine-Fe séparés à l'étape A1) et le Fe total à partir de l'échantillon (S1) par spectrométrie de masse ;
B) Détermination de la teneur en composés contenant du soufre (S) et du sélénium (Se) de l'échantillon (S1), comprenant les étapes suivantes :
B1) Séparation simultanée des composés contenant du S et du Se présents dans l'échantillon (S1) par électrophorèse capillaire ;
B2) Quantifier simultanément les composés contenant du soufre et du sélénium séparés à l'étape B1) de l'échantillon (S1) par spectrométrie de masse ;
les composés contenant du soufre étant choisis parmi le groupe constitué du glutathion oxydé (GSSG), du glutathion réduit (GSH), de la cystine et/ou de la cystéine ;
et les composés contenant du Se sont choisis parmi le Se⁴⁺ , le Se⁶⁺ , la Se-méthionine, la Se-cystéine GPX4, la sélénoprotéine P et/ou les sélénosaccharides.

2. Procédé d'analyse selon la revendication 1, dans lequel
I) à l'étape A1), l'échantillon est injecté à l'entrée ;
II) après l'injection de l'échantillon, le capillaire est traité avec un électrolyte (E2) à l'étape A1), l'électrolyte (E2) étant une solution aqueuse de HCl, de préférence d'une concentration comprise entre 0,005 et 0,2 mM, plus préférablement entre 0,01 et 0,1 mM, et de manière encore plus préférable de 0,05 mM ; et/ou
III) la séparation à l'étape A1) par électrophorèse capillaire comprend une étape de préparation du capillaire avant la séparation proprement dite, comprenant une purge et un remplissage avec un électrolyte de tête ainsi qu'un empilement, de préférence avec une solution (E1) comprenant du HCl et du MeOH ; dans lequel, éventuellement
i) la concentration en HCl est comprise entre 5 et 15 % en poids, de préférence entre 8 et 12 % en poids, plus préférablement 10 % en poids ; par rapport à la quantité totale de la solution et/ou
ii) la concentration en MeOH est comprise entre 5 et 20 % en poids, de préférence entre 7 et 15 % en poids, plus préférablement 10 % en poids, par rapport à la quantité totale de la solution et/ou
IV) l'électrolyte (E3) à l'entrée de l'étape A1) est une solution aqueuse de HCl, de préférence d'une concentration comprise entre 20 et 80 mM, de préférence encore entre 30 et 70 mM, et de préférence encore 50 mM ; et/ou
V) l'électrolyte (E4) de l'étape A1) à la sortie est une solution aqueuse d'HCl, de préférence d'une concentration comprise entre 1 et 15 mM, de préférence encore entre 3 et 8 mM, et de préférence encore 5 mM ; et/ou
VI) dans lequel la séparation à l'étape B1) par électrophorèse capillaire comprend une étape de préparation du capillaire avant la séparation proprement dite, comprenant le purgage et le remplissage avec l'électrolyte de tête (E5) ainsi que l'empilement d' , de préférence avec une solution comprenant un tampon borate et du CTAB (bromure de cétyltriméthylammonium), dans laquelle, éventuellement
i) la concentration en borate est comprise entre 50 et 200 mM, de préférence entre 80 et 150 mM, plus préférablement 90 mM ; et/ou
ii) la concentration en CTAB est comprise entre 1 et 10 mM, de préférence entre 1 et 5 mM, plus préférablement 3 mM ; et/ou
VII) l'électrolyte de l'étape B1) à l'entrée est une solution aqueuse (E6) comprenant du HCl, de préférence à une concentration comprise entre 5 et 100 mM, plus préférablement entre 10 et 50 mM, et de manière encore plus préférable à 20 mM ; et/ou
VIII) à l'étape B1), l'échantillon se trouve à l'entrée ; et/ou
IX) l'électrolyte (E7) de l'étape B1) à l'entrée est une solution aqueuse comprenant du TMAH (hydroxyde de tétraméthylammonium), de l'EtOH et/ou du CTAB (bromure de cétyltriméthylammonium), dans laquelle, éventuellement
i) la concentration en TMAH est comprise entre 0,5 et 10 % en poids, de préférence entre 1 et 5 % en poids, de manière encore plus préférentielle à 3 % en poids ; par rapport à la quantité totale de la solution aqueuse et/ou
ii) la concentration en EtOH est comprise entre 5 et 20 % en poids, de préférence entre 7 et 15 % en poids, et de manière encore plus préférentielle à 10 % en poids ; par rapport à la quantité totale de la solution aqueuse ; et/ou
iii) la concentration en CTAB est comprise entre 1 et 10 mM, de préférence entre 1 et 5 mM, et de manière encore plus préférentielle à 3 mM ; et/ou
X) l'électrolyte (E8) à la sortie de l'étape B2) est une solution aqueuse comprenant de l'acétate_{de} NH₄, de préférence à une concentration comprise entre 1 et 15 mM, de préférence encore entre 3 et 8 mM, et de manière encore plus préférentielle à 5 mM.

3. Procédé d'analyse selon la revendication 1 ou 2, dans lequel, à l'étape A2) et/ou B2), la quantification est réalisée par spectroscopie de masse à plasma à couplage inductif (ICP), la spectroscopie de masse à plasma à couplage inductif (ICP) étant, le cas échéant,
I) choisie parmi le groupe constitué de l'ICP-DRC-MS, de l'ICP-qqq-MS et de l'ICP-sf-MS, ou
II) l'ICP-DRC-MS, dans laquelle, éventuellement
i) à l'étape A2), le gaz DRC est _{du} NH₃ et/ou
ii) à l'étape B2), le gaz DRC est _{du} CH₄.

4. Procédé d'analyse selon l'une quelconque des revendications 1 à 3, dans lequel l'échantillon (S1) comprend une matière obtenue à partir d'un sujet, de préférence un sujet humain.

5. Procédé d'analyse selon l'une quelconque des revendications 1 à 4, dans lequel l'échantillon (S1) comprend un fluide biologique, de préférence choisi parmi le groupe constitué d'un lysat cellulaire, d'un lysat tissulaire, du LCR_{,} du sérum et de l'urine.

6. Procédé d'analyse selon l'une quelconque des revendications 1 à 5, dans lequel
I) le volume de l'échantillon (S1) n'est pas supérieur à 20 µl, de préférence pas supérieur à 15 µl, plus préférablement pas supérieur à 10 µl ; et/ou
II) le volume utilisé dans les étapes A1) et A2) combinées n'est pas supérieur à 30 nL, de préférence pas supérieur à 20 nL, et de manière encore plus préférée pas supérieur à 15 nL ; et/ou
III) le volume utilisé dans les étapes B1) et B2) combinées n'est pas supérieur à 30 nL, de préférence pas supérieur à 20 nL, et de manière encore plus préférable pas supérieur à 15 nL.

7. Procédé d'analyse selon l'une quelconque des revendications 1 à 6, dans lequel
I) les étapes A1) et A2) et/ou
II) les étapes B1) et B2)
sont réalisées en 1 à 15 minutes, de préférence en 3 à 10 minutes, plus préférablement en 4 à 7 minutes, et de manière encore plus préférable en moins de 5 minutes.

8. Procédé d'analyse selon l'une quelconque des revendications 1 à 7, dans lequel les étapes A) et B) sont réalisées séquentiellement, de préférence l'étape A) avant l'étape B).

9. Procédé d'analyse selon l'une quelconque des revendications 1 à 8, comprenant en outre la fourniture d'informations sur les résultats du procédé d'analyse, de préférence à un sujet.

10. Utilisation de la méthode d'analyse selon l'une quelconque des revendications 1 à 9 pour un diagnostic ex vivo du stress oxydatif.

11. Utilisation de la méthode d'analyse selon l'une quelconque des revendications 1 à 9 pour un diagnostic ex vivo de la ferroptose.

12. Utilisation de la méthode d'analyse selon l'une quelconque des revendications 1 à 9 pour le diagnostic ex vivo de maladies associées à la ferroptose, de préférence l'hémochromatose ou l'anémie sidéroblastique.

13. Utilisation de la méthode d'analyse selon l'une quelconque des revendications 1 à 9 pour le diagnostic ex vivo du cancer de la prostate, du mélanome, de la sclérose en plaques, du diabète et du syndrome myélodysplasique.

14. Kit destiné à mettre en œuvre la méthode d'analyse selon l'une quelconque des revendications 1 à 9, comprenant
i) des dispositifs permettant de mettre en œuvre la méthode d'analyse par électrophorèse capillaire couplée à la spectrométrie de masse ; et
ii) une solution de HCl servant d'électrolyte (E3) à l'entrée, de préférence à une concentration de 20 à 80 mM, de préférence encore de 30 à 70 mM, et de préférence encore de 50 mM ; pour réaliser l'électrophorèse capillaire (étape A1) et
iii) une solution de HCl servant d'électrolyte (E4) à la sortie, de préférence d'une concentration comprise entre 1 et 15 mM, de préférence encore entre 3 et 8 mM, et de préférence encore à 5 mM ; pour la mise en œuvre de l'électrophorèse capillaire (étape A1) et
iv) du gaz CH₄pour réaliser la spectrométrie de masse (étape B2).
